# EUROPEAN PATENT SPECIFICATION

(11) **EP 2 670 908 B1**
(45) Date of publication and mention of the grant of the patent: **04.04.2018**
(21) Application number: 12742220.2
(22) Date of filing: 02.02.2012
(51) Int. Cl.: D21C 11/14, C07C 29/80, C07C 31/04, D21C 11/00

(54) **METHOD OF MAKING PURIFIED METHANOL FROM A LIQUEFIED FRACTION OBTAINED FROM A SULFATE PULP PROCESS**
VERFAHREN ZUR HERSTELLUNG VON GEREINIGTEM METHANOL AUS EINER AUS EINEM SULFATMASSEVERFAHREN GEWONNENEN VERFLÜSSIGTEN FRAKTION
PROCÉDÉ DE FABRICATION DE MÉTHANOL PURIFIÉ À PARTIR D'UNE FRACTION LIQUÉFIÉE OBTENUE À PARTIR D'UN PROCÉDÉ DE FABRICATION DE PÂTE AU SULFATE

(30) Priority: 03.02.2011 FI 20115107
(43) Date of publication of application: 11.12.2013
(73) Proprietor: Stora Enso Oyj, 00101 Helsinki (FI)
(72) Inventor: RÄSÄNEN, Tiina, FI-55420 Imatra (FI); VILJAMAA, Katja, FI-33180 Tampere (FI); ISOAHO, Jukka Pekka, FI-40740 Jyväskylä (FI); ALÉN, Raimo, FI-00520 Helsinki (FI); PAKKANEN, Hannu, FI-40600 Jyväskylä (FI)
(74) Representative: Berggren Oy, Helsinki & Oulu
(86) International application number: PCT/FI2012/050100
(87) International publication number: WO 2012/104491

(56) References cited:
- WO-A1-97/35063
- WO-A1-97/35063
- WO-A1-2008/006190
- WO-A1-2009/012597
- WO-A1-2009/070110
- WO-A1-2010/091492
- WO-A2-2007/117481
- SE-B- 430 411
- US-A- 5 450 892
- US-A- 5 830 314
- US-A1- 2010 316 553
- DUFRESNE R. ET AL.: 'Treatment of Clean Condensate Using Catalytically Enhanced Oxidation' PROCEEDINGS OF THE 2000 TAPPI INTERNATIONAL ENVIRONMENTAL CONFERENCE pages 89 - 101, XP008171117

## Description

The present invention relates to a method for making refined methanol from condensate obtained from sulfate pulp cooking and containing methanol and sulfur contaminants, its main sulfur contaminants being methyl mercaptan, dimethyl sulfide and dimethyl disulfide.

Traditionally, methanol has been made by the dry distillation of wood, producing charcoal along with methanol. However, this methanol making process has become redundant because of its costliness and poor yield.

At present, methanol is manufactured industrially from a synthesis gas consisting of carbon monoxide and hydrogen. Wood is one of the suggested sources of synthesis gas, wherein wood is gasified with oxygen and the obtained gas is reformed catalytically and cleaned of contaminants to produce a mixture of carbon monoxide and hydrogen suitable for methanol synthesis.

In forest industry, methanol present in wood is also released in sulfate pulp cooking, ending up in the condensate condensed from cooking vapors. The main composition of condensate is > 50 weight-% (typically about 55 weight-%) of methanol, < 10 weight-% of ethanol, < 5 weight-% of acetone, about 10 weight-% of sulfur contaminants, and the balance (typically about 20 weight-%) is water. A typical composition of sulfur contaminants is in turn < 50 weight-% of methyl mercaptan, < 30 weight-% of dimethyl sulfide, < 20 weight-% of hydrogen sulfide, and < 10 weight-% of dimethyl disulfide.

A problem regarding the viable utilization of methanol present in sulfate cooking condensate is the effective separation of sulfur compounds from methanol. The distillation of condensate or water vapor distillation does not provide an efficient way of removing methanol from organic sulfur contaminants of the condensate. The boiling points of Methyl mercaptan and dimethyl sulfide are lower than those of methanol and should be separated by distillation prior to the distillation of methanol. Otherwise they would become distilled along with methanol. On the other hand, the boiling point of dimethyl disulfide is higher than that of methanol, being therefore left in the distillation residue in methanol distillation. The attainment of pure methanol would require several distillation cycles, making the refining process inconvenient and inefficient.

FI patent publication 52710 discloses a method for the purification of methanol separated from sulfate cooking condensate, comprising treating the condensate or impure methanol with sulfuric acid, distilling the methanol to remove it from water phase, oxidizing along-distilled sulfur impurities with hydrogen peroxide, and finally separating pure methanol with another distillation cycle. Hence, the question is about a multiphase process, which the Applicant does not find economically viable.

The removal of sulfur or sulfur compounds from alcohols, such as methanol or ethanol, has been described in publication US 2007/0238907 A1, wherein the sulfur compounds are adsorbed to the surface of a metal or metal alloy. Reference is made to Iron, copper and zinc as suitable metals. However, the sulfur compounds mentioned in the publication are solely inorganic. At least as such, the procedure cannot be expected to work for organic sulfur compounds.

For the above reasons, the sulfate cooking condensate has been mostly exploited by burning it in energy production. Being a sulfur-containing fuel, it has nevertheless been problematic in terms of environmental protection.

It is an object of the invention to provide a solution, whereby the removal of methanol from the low-boiling organic sulfur contaminants of sulfate cooking condensate and thereby also the production of pure methanol become efficient and economically feasible. Thus, an object of the invention is to enable the utilization of sulfate cooking condensate as a raw material in methanol production, such condensate having been regarded thus far as low value or even problematic. The invention is characterized in that the condensate is subjected to an oxidation treatment, wherein methyl mercaptan and dimethyl sulfide, which have boiling points lower than that of methanol, are oxidized to higher-boiling dimethyl sulfoxide and dimethyl sulfone, followed by removing the methanol by distillation or water vapor distillation from distillation residue containing the sulfur-bearing oxidation products having boiling points higher than that of methanol.

The invention is based on the surprising discovery that it is possible to oxidize sulfur compounds of low boiling point, such as methyl mercaptan (CH₃-S-H) and dimethyl sulfide (CH₃-S-CH₃), into higher boiling sulfur compounds without having the present methanol oxidized at the same time. The removal of methanol can be subsequently conducted by distillation or water vapor distillation from sulfur-containing oxidation products, which are left in distillation residue. The oxidation products include dimethyl sulfoxide (CH₃-SO-CH₃) and dimethyl sulfone (CH₃-SO₂-CH₃), which are isolable from distillation residue and of which particularly dimethyl sulfone has value as a commercial product. The latter aspect is also a contributing factor to viability of the process as a way of making pure methanol.

Another major benefit of the invention is avoiding the use of sulfur-containing condensate as a fuel and the environmental problems resulting therefrom.

The oxidation reactions of dimethyl sulfide into dimethyl sulfoxide or dimethyl sulfone are prior known as such. The oxidation of dimethyl sulfide with nitrogen tetroxide in water-free conditions is a known process of making dimethyl sulfoxide. The oxidation of sulfur compounds, such as hydrogen sulfide, methyl mercaptan and dimethyl sulfide, with hydrogen peroxide in waste water purification has been described in the article Feliers, C., Patria, L., Morvan, J., Laplanche, A., Environmental Technology, Vol. 22, 2001, pp. 1137-1146*.* The purpose is to reduce odor nuisances caused by sulfur compounds. The oxidation of dimethyl sulfide into dimethyl sulfoxide and dimethyl sulfone as an atmospheric photochemical reaction has been described, among others, in the article Yin, F., Grosjean, D., Flagan, R.C., Seinfeld, J.H., Journal of Atmospheric Chemistry II, 1990, pp. 365-399*.* None of these publications nevertheless discloses the oxidation of organic sulfur compounds, with methanol serving as a main liquid medium for the reaction. Oxidants suitable for a purification process of the invention are peroxides, oxygen, ozone, chlorine, permanganate or hypochlorite, especially hydrogen peroxide.

The oxidation of sulfur compounds takes place in the invention most preferably in the presence of a metal-containing catalyst. The metal contained in the catalyst can be e.g. Fe, Cu or Zn, and it can be in its elemental state or in the form of a compound, such as oxide or sulfate. After the oxidation process, prior to distillation of methanol or water vapor distillation, the catalyst can be separated from liquid phase by filtration.

A preferred way of applying the invention is such that the condensate is first subjected to oxidation with hydrogen peroxide or the like suitable oxidant, preferably in the presence of a metal or metal compound catalyst. The catalyst is removed from the liquid reaction mixture by filtration, after which pure methanol is separated by distillation or water vapor distillation while sulfur-containing oxidation products are left in distillation residue. The aqueous distillation residue is exploitable preferably by crystallizing dimethyl sulfone therefrom as a valuable byproduct of the process.

In oxidation, dimethyl sulfide oxidizes first into dimethyl sulfoxide, which is a fast exothermic reaction. Conversely, the oxidation of dimethyl sulfoxide in the next stage into dimethyl sulfone is a slow reaction, which nevertheless speeds up substantially by heating the reaction medium. Should the separation of methanol from reaction medium be commenced before the oxidation is completed, conveniently while the second process stage is still in progress, the distillation brings heat into the reaction medium, thus preferably accelerating at the same time the formation of dimethyl sulfone and thereby the completion of the process. Hence, whenever necessary, the supply of oxidant into the reaction mixture can also be continued even during the course of distillation.

The following working examples describe the oxidation of sulfate cooking condensate, which is a critical stage of the process. A successful oxidation converts the low-boiling sulfur contaminants, methyl mercaptan and dimethyl sulfide, into dimethyl sulfoxide and further into dimethyl sulfone, whereby pure methanol is capable of being distilled or water vapor distilled to remove it from distillation residue which retains the oxidized sulfur compounds.

### Example 1

For the trials, in order to ensure the comparability of results, there was prepared a synthetic reference condensate with a constant composition of methanol (67,3 volume-%), water (19,2 volume-%), ethanol (6,7 volume-%), and acetone (2,9 volume-%), as well as sulfur compounds dimethyl sulfide (2,9 volume-%) and dimethyl disulfide (1,0 volume-%). 25 mL of this condensate (pH 7,5) was measured into an Erlenmeyer (100 mL), to which was then added 0,72 g of metallic iron as a catalyst and 6 mL of 30 volume-% hydrogen peroxide as an oxidant. The resulting mixture was stirred at room temperature for one hour, allowed to stabilize for 10 minutes without stirring, and filtered (filter's pore size 0,45 µm) for removing the catalyst. In order to demonstrate the oxidation product's crystallization effect, the mixture's methanol was allowed to evaporate gradually at room temperature at normal pressure, thus producing white crystals, which by a detailed X-ray diffraction analysis (D.E. Sands; Z. Kristallogr., Kristallgeom., Kristallphys., Kristallchem., 119(1963)245, D.A. Langs, J.V. Silverton and W.M. Bright; J. Chem. Soc. D., (1970)1653) were found to consist totally of pure dimethyl sulfone. On the other hand, the overall sulfur budget based on X-ray fluorescence measurement showed that the total yield of crystallized dimethyl sulfone was about 85 weight-% as calculated from initial sulfur. The negligible presence of inorganic sulfur compounds was confirmed ion chromatographically.

### Example 2

The oxidation of synthetic condensate was conducted as in example 1, the only difference being that the condensate had no addition of metal catalyst. Even in this case, the overall yield of dimethyl sulfone was found to remain at the level of 85 weight-%.

### Example 3

The oxidations of synthetic condensate were conducted as in examples 1 and 2 with the difference that, prior to the oxidation, the condensate had its pH adjusted with an aqueous solution of 25% sodium hydroxide to the value of 12. Even now, the total yield of dimethyl sulfone was about 85 weight-% in trials conducted both with catalysts and without a catalyst.

In order to simplify the conduction of laboratory trials, methanol has been allowed in the above examples 1-3 to separate by evaporation, but it is obvious for a skilled artisan that methanol is equally removable by distillation or water vapor distillation. The oxidized sulfur compounds, as well as water present in the reaction mixture, all have boiling points higher than that of methanol and remain in distillation residue.

## Claims

1. A method for making refined methanol from condensate obtained from sulfate pulp cooking and containing methanol and sulfur contaminants, its main sulfur contaminants being methyl mercaptan, dimethyl sulfide and dimethyl disulfide, **characterized in that** the condensate is subjected to an oxidation treatment, wherein methyl mercaptan and dimethyl sulfide, which have boiling points lower than that of methanol, are oxidized to higher-boiling dimethyl sulfoxide and dimethyl sulfone, followed by removing the methanol by distillation or water vapor distillation from distillation residue containing the sulfur-bearing oxidation products having boiling points higher than that of methanol.

2. A method as set forth in claim 1, **characterized in that** the oxidant is peroxide, oxygen, ozone, chlorine, permanganate or hypochlorite, preferably hydrogen peroxide.

3. A method as set forth in claim 1 or 2, **characterized in that** the oxidation takes place in the presence of a metal-containing catalyst.

4. A method as set forth in claim 3, **characterized in that** the distillation or water vapor distillation of methanol is preceded by removing the catalyst therefrom by filtration.

5. A method as set forth in claim 3 or 4, **characterized in that** the metal contained in the catalyst is Fe, Cu or Zn.

6. A method as set forth in any of claims 3-5, **characterized in that** the catalyst has its metal in elemental state or in the form of a compound, such as oxide or sulfate.

7. A method as set forth in any of the preceding claims, **characterized in that**, during the course of methanol removal conducted by distillation or water vapor distillation, the reaction medium is supplied with an oxidant, such as hydrogen peroxide, oxygen or air, for carrying the oxidation to completion.

8. A method as set forth in any of the preceding claims, **characterized in that**, the liquefied fraction forming the starting material contains > 50 weight-% of methanol, 2-10 weight-% of ethanol, 1-5 weight-% of acetone, and 5-15 weight-% of sulfur contaminants, the balance substantially consisting of water.

9. A method as set forth in claim 8, **characterized in that** the sulfur contaminants substantially consist of methyl mercaptan, dimethyl sulfide, hydrogen sulfide and dimethyl disulfide.

## Patentansprüche

1. Verfahren zum Herstellen von raffiniertem Methanol aus Kondensat, das aus Sulfat-Zellstoffkochung erhalten wird und das Methanol und Schwefelverunreinigungen enthält, dessen Hauptschwefelverunreinigungen Methylmercaptan, Dimethylsulfid und Dimethyldisulfid sind, **dadurch gekennzeichnet, dass** das Kondensat einer Oxidationsbehandlung ausgesetzt wird, wobei Methylmercaptan und Dimethylsulfid, die niedrigere Siedepunkte als Methanol haben, zu höhersiedenden Dimethylsulfoxid und Dimethylsulfon oxidiert werden, gefolgt von einem Entfernen des Methanols durch Destillation oder Wasserdampfdestillation aus Destillationsrückstand, der die schwefelhaltigen Oxidationsprodukte mit höheren Siedepunkten als Methanol enthält.

2. Verfahren nach Anspruch 1, **dadurch gekennzeichnet, dass** das Oxidationsmittel Peroxid, Sauerstoff, Ozon, Chlor, Permanganat oder Hypochlorit, vorzugsweise Wasserstoffperoxid ist.

3. Verfahren nach Anspruch 1 oder 2, **dadurch gekennzeichnet, dass** die Oxidation in Anwesenheit eines Metall enthaltenden Katalysators stattfindet.

4. Verfahren nach Anspruch 3, **dadurch gekennzeichnet, dass** der Destillation oder Wasserdampfdestillation von Methanol ein Entfernen des Katalysators davon durch Filtration vorangestellt ist.

5. Verfahren nach Anspruch 3 oder 4, **dadurch gekennzeichnet, dass** das Metall, das in dem Katalysator enthalten ist, Fe, Cu oder Zn ist.

6. Verfahren nach einem der Ansprüche 3-5, **dadurch gekennzeichnet, dass** der Katalysator sein Metall in elementarem Zustand oder in Form einer Verbindung, wie etwa Oxid oder Sulfat, aufweist.

7. Verfahren nach einem der vorhergehenden Ansprüche, **dadurch gekennzeichnet, dass** im Laufe der Methanolentfernung, die durch Destillation oder Wasserdampfdestillation durchgeführt wird, dem Reaktionsmedium ein Oxidationsmittel, wie etwa Wasserstoffperoxid, Sauerstoff oder Luft, zum Abschließen der Oxidation zugeführt wird.

8. Verfahren nach einem der vorhergehenden Ansprüche, **dadurch gekennzeichnet, dass** der verflüssigte Anteil, der das Ausgangsmaterial bildet, > 50 Gewichts-% Methanol, 2-10 Gewichts-% Ethanol, 1-5 Gewichts-% Aceton und 5-15 Gewichts-% Schwefelverunreinigungen enthält, wobei das Gleichgewicht im Wesentlichen aus Wasser besteht.

9. Verfahren nach Anspruch 8, **dadurch gekennzeichnet, dass** die Schwefelverunreinigungen im Wesentlichen aus Methylmercaptan, Dimethylsulfid, Wasserstoffsulfid und Dimethyldisulfid bestehen.

## Revendications

1. Procédé de production de méthanol raffiné à partir de condensat obtenu en cuisant de la pulpe de sulfate et contenant des contaminants au méthanol et au soufre, ses contaminants principaux au soufre étant du méthylmercaptane, diméthylsulfure et diméthyldisulfure, **caractérisé en ce que** le condensat est soumis à un traitement par oxydation, le méthylmercaptane et le diméthylsulfure, qui ont des points d'ébullition inférieurs à celui du méthanol, sont oxydés en diméthylsulfoxyde et en diméthylsulfone qui pont des points d'ébullition plus faibles que celui du méthanol sont oxydés en diméthylsulfoxyde et en diméthylsulfone à point d'ébullition plus élevé, ce qui est suivi de l'élimination du méthanol par distillation ou distillation de vapeur d'eau à partir de résidus de distillation contenant les produits d'oxydation porteurs de soufre ayant des points d'ébullition plus élevés que celui du méthanol.

2. Procédé selon la revendication 1, **caractérisé en ce que** l'oxydant est du peroxyde, de l'oxygène, de l'ozone, du chlore, du permanganate ou de l'hypochlorite, de préférence du peroxyde d'hydrogène.

3. Procédé selon la revendication 1 ou 2, **caractérisé en ce que** l'oxydation a lieu en présence d'un catalyseur contenant du métal.

4. Procédé selon la revendication 3, **caractérisé en ce que** la distillation ou la distillation de vapeur d'eau du méthanol est précédée par l'élimination du catalyseur de celui-ci par filtration.

5. Procédé selon la revendication 3 ou 4, **caractérisé en ce que** le méthane contenu dans le catalyseur est du Fe, Cu ou Zn.

6. Procédé selon l'une quelconque des revendications 3 à 5, **caractérisé en ce que** le catalyseur comporte son métal au stade élémentaire ou sous la forme d'un composé comme de l'oxyde ou du sulfate.

7. Procédé selon l'une quelconque des revendications précédentes, **caractérisé en ce que**, au cours de l'élimination du méthanol réalisée par distillation ou distillation de vapeur d'eau, le milieu réactif est alimenté en oxydant comme du peroxyde d'hydrogène, de l'oxygène ou de l'air pour amener l'oxydation à son terme.

8. Procédé selon l'une quelconque des revendications précédentes, **caractérisé en ce que** la fraction liquéfiée formant la matière initiale contient > 50 % en poids de méthanol, 2 à 10 en poids d'éthanol, 1 à 5 en poids d'acétone et 5 à 15 de contaminants au soufre, le solde étant substantiellement composé d'eau.

9. Procédé selon la revendication 8, **caractérisé en ce que** les contaminants au soufre sont composés substantiellement de méthylmercaptane, diméthylsulfure, sulfure d'hydrogène et diméthyldisulfure.
